(19) **Europäisches Patentamt**

**European Patent Office**

**Office européen des brevets**

(11) **EP 0 904 357 B1**

(12) **EUROPÄISCHE PATENTSCHRIFT**

(45) Veröffentlichungstag und Bekanntmachung des Hinweises auf die Patenterteilung:
**13.12.2006 Patentblatt 2006/50**

(21) Anmeldenummer: **98904046.4**

(22) Anmeldetag: **08.01.1998**

(51) Int Cl.:
*C12N 9/16* (2006.01)     *C12N 9/18* (2006.01)
*C12N 1/15* (2006.01)     *C12P 21/02* (2006.01)
*C11B 3/00* (2006.01)

(86) Internationale Anmeldenummer:
**PCT/EP1998/000081**

(87) Internationale Veröffentlichungsnummer:
**WO 1998/031790 (23.07.1998 Gazette 1998/29)**

(54) **PROTEIN MIT PHOSPHOLIPASEAKTIVITÄT**

PROTEIN WITH PHOSPHOLIPASE ACTIVITY

PROTEINE AVEC ACTIVITE DE PHOSPHOLIPASE

(84) Benannte Vertragsstaaten:
**AT BE CH DE DK ES FI FR GB IE IT LI NL PT SE**

(30) Priorität: **16.01.1997 DE 19701348**

(43) Veröffentlichungstag der Anmeldung:
**31.03.1999 Patentblatt 1999/13**

(73) Patentinhaber: **AB Enzymes GmbH**
**64293 Darmstadt (DE)**

(72) Erfinder:
• **LÖFFLER, Fridolin**
**D-64625 Bensheim (DE)**
• **JUNGSCHAFFER, Gerald**
**D-64665 Alsbach-Hähnlein (DE)**
• **KHANH, Quoc, Nguyen**
**D-64385 Reichelsheim (DE)**

• **SCHUSTER, Erwin**
**D-64625 Bensheim-Auerbach (DE)**
• **SPRÖSSLER, Bruno**
**D-64380 Rossdorf (DE)**
• **WOLF, Sabine**
**D-64853 Otzberg (DE)**

(74) Vertreter: **Hiebl, Inge Elisabeth et al**
**Kraus & Weisert**
**Patent- und Rechtsanwälte**
**Thomas-Wimmer-Ring 15**
**80539 München (DE)**

(56) Entgegenhaltungen:
**EP-A- 0 575 133**      **EP-A- 0 808 903**
**WO-A-95/22615**      **WO-A-97/05219**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Protein mit Phospholipaseaktivität, erhältlich durch Spalten der reifen Sequenz der Aspergillus-Lysophospholipase mit SEQ ID NO: 2 in zwei Spaltstücke, wobei die Spaltstücke über mindestens eine unter reduzierenden Bedingungen spaltbare Bindung verknüpft sind oder wobei von den unverknüpften Spaltstücken mindestens eines Phospholipaseaktivität besitzt. Ferner betrifft die Erfindung ein Verfahren zur Produktion dieses Proteins sowie die Verwendung dieses Proteins zur Entschleimung von pflanzlichen Ölen und als Backhilfsmittel.

Bei der Entschleimung von Speiseöl werden nicht hydratisierbare Phospholipide durch Phopholipase wasserlöslich gemacht und so schonend, kostensparend und umweltfreundlich aus dem Speiseöl entfernt. In der europäischen Patentanmeldung 0 513 709 (Röhm/Lurgi) wird erstmals ein wirksames enzymatisches Verfahren zur Entschleimung vorgestellt. Dabei wird ein mit Wasser vorentschleimtes Speiseöl mit einer wäßrigen Lösung einer Phospholipase zu Tröpfchen kleiner 10 μm emulgiert. Nach der Hydrolyse (pH 3 bis 6, Temperatur 50 bis 70°C) wird die wäßrige Phase abgetrennt. Das enzymatische Entschleimungsverfahren wurde als "EnzyMax-Verfahren" von der Firma Lurgi in der Speiseölindustrie eingeführt. In der DE 43 39 556 wird als weitere Variante dieses Verfahrens die Wiederverwendung des Enzyms beschrieben, indem man das Enzym aus einer gebrauchten, schlammhaltigen wäßrigen Phase durch Zusatz von Tensiden oder Lösungsvermittlern ablöst und als weitgehend schlammfreie, enzymhaltige Lösung wiederverwendet.

Die Bereitstellung der erforderlichen Mengen an Enzym für die Betreibung eines großtechnischen Verfahrens kann nur mit Hilfe von Mikroorganismen gedeckt werden. Es besteht also ein Bedarf nach einer mikrobiellen Quelle, die erlaubt, das Enzym Phospholipase in unbeschränkten Mengen zu produzieren. In der DE-OS 195 27 274.9 vom 26.07.1995 (Röhm/Lurgi) wird beschrieben, daß in *Aspergillus niger* eine geeignete Phospholipase gefunden wurde. Sie spaltet Lecithin zu Lysolecithin, ist aber auch in der Lage Lysolecithin weiter zu spalten zum Phosphatidylcholin. Reine Lysophospholipasen aus *Aspergillus*, die nur Lysolecithin zu spalten vermögen, sind im Entschleimungsprozeß wirkungslos. Das gilt auch für die nicht acylspaltenden Phospholipasen C und D.

Ferner können Phospholipasen auch als Backhilfsmittel zur Verbesserung der Verarbeitung des Teigs verwendet werden.

Die EP-A-0 808 903 betrifft eine aus *Aspergillus* isolierbare rekombinante Desoxyribonukleinsäure, die eine Lysophospholipase codiert und eine definierte Nukleotidsequenz aufweist.

Die WO 97/05219 betrifft ein enzymatisches Verfahren zur Entschleimung von pflanzlichen Ölen mit *Aspergillus*-Phospholipase.

Die EP-A-0 575 133 beschreibt eine neue Phospholipase, die ein Phospholipid unter Bildung eines 2-Acylphospholipids hydrolisieren kann.

Die WO 95/22615 betrifft ein Verfahren zur Herstellung eines lipolytischen Enzyms sowie Varianten davon, die eine verminderte Abhängigkeit von Kalzium und/oder eine verbesserte Toleranz gegenüber Detergenzien besitzen.

Der vorliegenden Erfindung liegt die Aufgabe zugrunde, eine kostengünstige Phospholipase in hoher Reinheit herzustellen. Die Phospholipase soll durch einen transformierten Wirtsorganismus in großen Mengen produziert werden können. Mit dem Enzym sollen Präparate hergestellt werden können, die sich besonders gut zur Hydrolyse von Phospholipiden und somit zur Klärung von Stärkehydrolysaten und zur Herstellung von Backhilfsmitteln eignen.

Erfindungsgemäß wird diese Aufgabe gelöst durch ein Protein mit Phospholipaseaktivität, erhältlich durch Spalten der reifen Sequenz der Aspergillus-Lysophospholipase mit SEQ ID NO: 2 in zwei Spaltstücke, wobei die Spaltstücke entweder über mindestens eine unter reduzierenden Bedingungen spaltbare Bindung verknüpft sind oder wobei von den unverknüpften Spaltstücken mindestens eines Phospholipaseaktivität besitzt. Ferner wird diese Aufgabe gelöst durch ein Protein mit Phospholipaseaktivität, das dadurch gekennzeichnet ist, daß es von einem Antikörper gegen gereinigte Phospholipase aus *Aspergillus foetidus* RH 3046 erkannt wird.

Es wurde überraschenderweise gefunden, daß ein mit der aus *Aspergillus* isolierbaren Desoxyribonukleinsäure (DNA) gemäß der DE-OS 196 20 649.9 transformierter Mikroorganismus nicht nur eine Lysophospholipase codiert, sondern unter bestimmten Kulturbedingungen auch in eine Phospholipase prozessiert wird. Die Phospholipase besitzt somit die gleiche Primärstruktur wie die Lysophospholipase, jedoch eine andere Sekundär- und Tertiärstruktur und somit andere physiologische Eigenschaften. Die entsprechende Sequenz ist in SEQ ID No. 1 der DE-OS 196 20 649.9 dargestellt. Eine weitere Phospholipase codierende Sequenz wurde aus *Aspergillus niger* isoliert, sie unterscheidet sich in nur 6% der Aminosäuren von der homologen Sequenz aus *Aspergillus foetidus*. Sowohl die Phospholipase aus *Aspergillus niger* wie die Lysophospholipase aus *Aspergillus foetidus* bestehen aus 270 Aminosäuren und haben ein Molekulargewicht von 36000 Da (siehe SEQ ID No. 1+2). Bezüglich des Erhalts der transformierten Mikroorganismen wird auf die Offenbarung der DE-OS 196 20 649.9 Bezug genommen. Eine Phospholipase aus *Aspergillus* wurde bisher im Stand der Technik nicht beschrieben. Die Druckschrift Nakaya et al., Eur. J. Biochem. 1990, 193 (1) 31-38 beschreibt ein Protein, dessen Sequenz der Phospholipase A2 ähnlich ist.

Durch proteinchemische Methoden ließ sich Phospholipase von Lysophospholipase trennen und hochgereinigt gewinnen. Beim Vergleich der gereinigten Phospholipase und Lysophospholipase ergaben sich folgende Unterschiede:

- Die Molekulargewichte von Phospholipase und Lysophospholipase aus *Aspergillus foetidus*, gemessen mit der SDS-Gelelektrophorese, betragen unter reduzierenden Bedingungen ca. 30000 Da für Phospholipase und ca. 36000 Da für Lysophospholipase, unter nicht reduzierenden Bedingungen dagegen liegen sie für beide Enzyme gleich bei ca. 36000 Da. Unter reduzierenden Bedingungen zerfällt die Phospholipase in zwei Ketten, von denen die größere (30000 Da) im Elektrophoresegel erfaßt wird. Das Teilstück in der Größe von ca. 6000 Da kann aus methodischen Gründen im gleichen Elektrophoresegel nicht nachgewiesen werden, jedoch kann man aus diesem Befund ableiten, daß Phospholipase aus zwei Peptidketten besteht. Diese Vorstellung wird durch das Ergebnis der Proteinsequenzierung bestätigt.

- Die Proteinsequenzierung der Phospholipase aus *Aspergillus foetidus* ergab eine hohe Übereinstimmung mit der Sequenz der Lysophospholipase, jedoch auch Unterschiede. Bei Phospholipase wurden zwei $NH_2$-Termini im Verhältnis 1:1 gefunden, bei Lysophospholipase dagegen nur einer. Einer der beiden $NH_2$-Termini der Phospholipase gehört zu einem 6000 Da-Peptid, während der andere der $NH_2$-Terminus des 30000 Da-Proteins ist. Während das kleinere Peptid mit den Aminosäuren 1 bis 44 des reifen Lysophospholipase-Proteins (vgl. Sequenz ID No. 1 in DE-OS 196 20 649.9) übereinstimmt, entspricht die Sequenz des 30000 Da-Proteins den Aminosäuren 45 bis 270 der Lysophospholipase (vgl. Sequenz ID No. 1 in DE-OS 196 20 649.9).

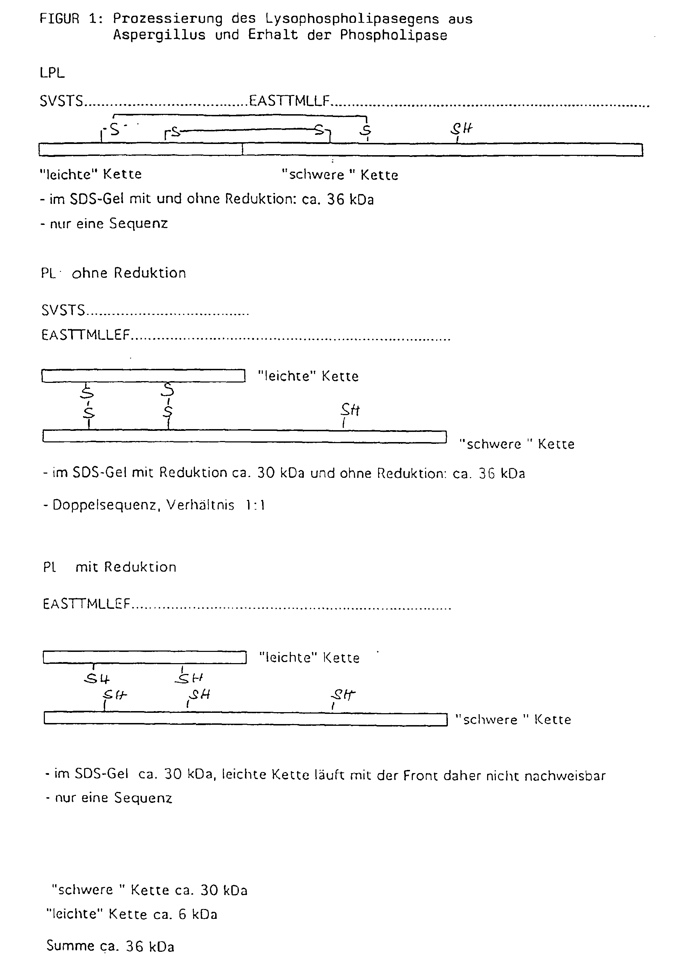

Dieser Befund legt nahe, daß die Phospholipase aus *Aspergillus foetidus* durch Prozessierung des Lysophospholipase-Proteins entstehen kann, wobei noch ungeklärt ist, ob die Prozessierung innerhalb oder außerhalb der Zelle stattfindet und in welcher Weise sie abläuft. Die Beziehungen zwischen Phospholipase und Lysophospholipase sind in Figur 1 dargestellt.

Weiter unterscheiden sich Phospholipase und Lysophospholipase in ihren isoelektrischen Punkten, ihren pH- und Temperaturoptima sowie sehr deutlich in ihren Temperaturstabilitäten. In der folgenden Tabelle werden diese Werte gegenübergestellt.

Tabelle 1: Vergleich der Eigenschaften von Phospholipase und Lysophospholipase aus *Aspergillus foetidus*

|  | Phospholipase | Lysophospholipase |
| --- | --- | --- |
| Molgewicht (SDS-Gel, reduz.) | 30000 Da | 36000 Da |
| Molgewicht (SDS-Gel, nicht-reduz.) | 36000 Da | 36000 Da |
| Isoelektr. Punkt | pH 4,3 | pH 4,2 |
| Temperatur-Optimum | 50°C | 55°C |
| pH-Optimum | pH 3-4 | pH 4,5 |
| pH-Stabilität (1 Std. bei 60°C) | pH 3,5 | pH 4,5 |
|  | >75% Restakt. | 10% Restakt. |

Zum Erhalt der Phospholipase an Stelle der Lysophospholipase aus den betreffenden Mikroorganismen sind die Fermentationsbedingungen wesentlich. Wesentlich für die Bildung der Phospholipase ist die Durchführung der Kultur in einem sauren bis leicht alkalischen Medium. Der pH-Wert liegt dabei geeigneterweise in einem Bereich von 2 bis 9, bevorzugt 3 bis 8. Unter diesen Bedingungen wird bevorzugt Phospholipase gebildet. Dabei wird wie folgt vorgegangen:

Zunächst wird ein geeigneter Wirt ausgewählt mit dem Ziel einer möglichst einfachen Herstellung der Phospholipase. Obwohl viele Arten von Schimmelpilzen als mögliche Wirte in Betracht kommen, wie zum Beispiel Vertreter der thermophilen Gattungen Humicola, Thermomyces und Mucor, der Gattungen Rhizopus, Absidia, Chaetomium, Trichoderma, Thielavia, Penicillium und Cephalosporium, wurden bevorzugt Arten der Gattung *Aspergillus* verwendet. Nach Transformation mit den erfindungsgemäßen Plasmiden lassen sich Transformanten isolieren, die, verglichen mit den Wirten, Phospholipase in großen Mengen produzieren. Bevorzugt ist der transformierte Wirtsorganismus ein Aspergillus-Stamm der Arten *Aspergillus oryzae, Aspergillus sojae, Aspergillus niger,* Aspergillus *awamori, Aspergillus foetidus, Aspergillus* ellipticus, *Aspergillus aculeatus, Aspergillus carbonarius* oder *Aspergillus phoenicis* oder ein Trichoderma-Stamm der Arten *Trichoderma viride, Trichoderma longibrachiatum* oder *Trichoderma reesei.*

Eine Transformante, die durch Cotransformation eines Wirtsstammes mit einem Selektionsplasmid, bevorzugt mit pAN7-1, p3SR2 oder pSTA10, und einem Expressionsplasmid, bevorzugt mit pKC3, pKC9, pKC12 oder pKCN2, hergestellt wird, züchtet man in einer für den Wirtsstamm üblichen Nährlösung, die mindestens eine verwertbare C-Quelle, wie z.B. Maisschrot, Stärke, Dextrin aus Stärke, und mindestens eine verwertbare organische N-Quelle, wie z.B. Maisquellwasser, Hefeautolysat, Sojamehl, Sojaprotein oder -pepton allein oder in Kombination mit anorganischen N-Quellen wie Ammoniumsalzen oder Nitraten, enthält und nach der Sterilisation auf einen sauren bis leicht alkalischen pH-Wert eingestellt wird. Die Nährlösung kann durch Zugabe von Substanzen, die in besonderem Maße die

Bildung der Phospholipase erhöhen, ergänzt werden. Phospholipide aus Soja enthalten solche Substanzen, doch kommen sie auch in anderen Stoffklassen, wie z.B. den Polyoxyethylenethern vor. Nach Beimpfen der sterilisierten Nährlösung mit Konidien oder vegetativem Mycel der Transformante wächst diese unter Belüftung bei Temperaturen zwischen 20° und 60°C, vorzugsweise zwischen 25° und 45°C, und produziert die erfindungsgemäße Phospholipase. Während der Kultivierung wird der pH-Wert der Kultur durch Säure- oder Laugezugabe korrigiert, so daß er im sauren bis schwach alkalischen Bereich, vorzugsweise zwischen pH 3 und 8, gehalten wird. Nach 48 bis 120 Stunden Kulturdauer kann man die Phospholipase gewinnen, indem man die unlöslichen Nährlösungsreste und die Biomasse abtrennt, was üblicherweise durch Filtration geschieht, und das Filtrat mit üblichen Methoden, wie z.B. durch Ultrafiltration, konzentriert. Das Konzentrat (Retentat) kann zur Entschleimung von Pflanzenölen oder zur Behandlung von Phospholipiden eingesetzt werden. Ferner kann die Phospholipase auch zur Verbesserung der rheologischen Eigenschaften von Lebensmitteln verwendet werden.

Die folgenden Mikroorganismen wurden bei der Deutschen Sammlung von Mikroorganismen und Zellkulturen (DSM), Mascheroder Weg 1B, 38124 Braunschweig, Deutschland, gemäß den Bestimmungen des Budapester Vertrages hinterlegt:

*A. oryzae* RH 3745: Hinterlegungsnummer DSM 11283
(Hinterlegungsdatum: 11.11.1996)

*A. ellipticus* RH 3886: Hinterlegungsnummer DSM 11284
(Hinterlegungsdatum: 11.11.1996)

*A. foetidus* RH 3046: Hinterlegungsnummer DSM 10652
(Hinterlegungsdatum: 24.04.1996)
*E. coli* DH5$\alpha$ pKC3: Hinterlegungsnummer DSM 10653
(Hinterlegungsdatum: 24.04.1996)
*E. coli* DH5$\alpha$ pKC9: Hinterlegungsnummer DSM 10654
(Hinterlegungsdatum: 24.04.1996)
*E. coli* DH5$\alpha$ pKC12: Hinterlegungsnummer DSM 10655
(Hinterlegungsdatum: 24.04.1996)
*E. coli* pKCN2: Hinterlegungsnummer: DSM 11352
(Hinterlegungsdatum: 23.12.1996)
*A. niger* RH 3909: Hinterlegungsnummer DSM 11353
(Hinterlegungsdatum: 23.12.1996)

Die Erfindung wird anhand der nachstehenden Beispiele und Figuren näher erläutert.

Die Figur 1 zeigt die Prozessierung des Lysophospholipasegens aus *Aspergillus* und den Erhalt der Phospholipase.

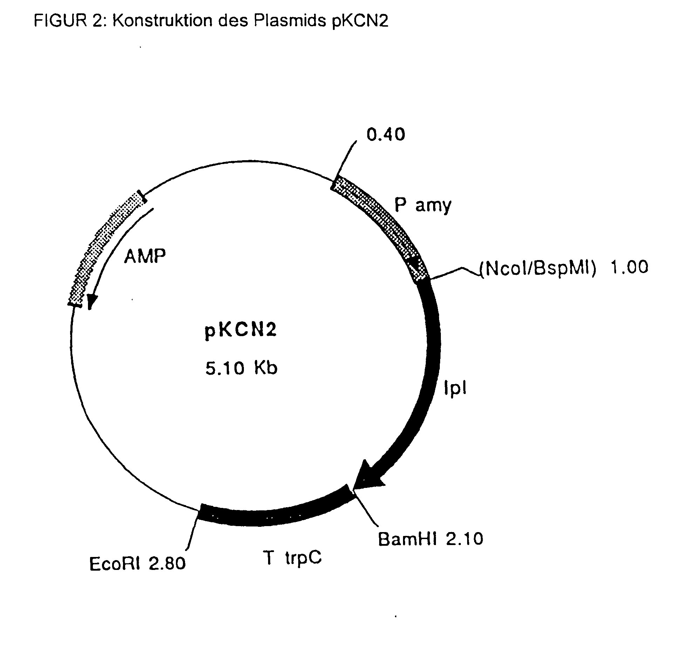

Die Figur 2 zeigt die Konstruktion des Plasmids pKCN2.

**Beispiele**

Beispiel 1

Konstruktion des Expressionsvektors pKCN2

Isolierung des Lysophospholipase-Gens aus *A. niger* NRRL3

Die chromosomale DNA von *A. niger* NRRL3 wurde nach einer Vorschrift von Hynes, M.J. et al. (1983), Mol. Cell. Biol. 3, 1430-1439, isoliert.

Die erhaltene hochmolekulare DNA wurde mit *Sau*3AI partiell hydrolysiert und durch Saccharose-Dichtegradientenzentrifugation nach ihrer Größe fraktioniert. DNA-Moleküle der Größe von 9 bis 20 kb wurden in *Bam*HI/*Eco*RIhydrolysierte EMBL3-DNA insertiert und in-vitro verpackt.

Zur Identifizierung des chromosomalen Lysophospholipase-Gens in einer Lambda EMBL3-Genbank wurde das *Hind*III/*Sal*I-cDNA Fragment aus dem Plasmid pKCl/36 als Hybridisierungssonde verwendet. Das Plasmid pKCl/36 enthielt die aus *A. foetidus* RH 3046 isolierte Lysophospholipase-cDNA.

Nach der Hybridisierung und mehrfacher Vereinzelung ließen sich zwei positive Klone identifizieren. Die Phagen-DNA von Klon Nr. 1 wurde präpariert und mit *Bam*HI hydrolysiert. Sie wies nach der Southern-Hybridisierung ein positives Signal bei ca. 9 kb auf. Das *Bam*HI-Fragment wurde in pUC18 kloniert, und das resultierende Plasmid, welches das vollständige chromosomale Lysophospholipase-Gen enthielt, wurde als pKCN bezeichnet.

Konstruktion des Expressionsvektors pKCN2

In das Plasmid pKCN2 wurde das Lysophospholipase-Gen unter Kontrolle des *A. oryzae* alpha-Amylase-Promotors und des *A. nidulans trp*C-Terminators gesetzt.

Die Isolierung des Lysophospholipase-Gens aus dem Plasmid pKCN erfolgte mit Hilfe der PCR-Methode. Es wurden zwei Oligonukleotid-Primer mit den folgenden Sequenzen verwendet:

KC29:

```
5'-GGA ATT CAC CTG CTA ACC ATG TTC TCT GGA CGG TTT GGA GTG-3'
              BspMI           Met
(SEQ ID No. 3)
```

KC43:

```
5'-CG GGATCC AAG CTA TAG CAG ACA CTC TGA AAT TG-3'
      BamHI           AMB
(SEQ ID No. 4)
```

Für die Polymerase-Ketten-Reaktion wurden in einem Reaktionsvolumen von 0,1 ml 20 mM Tris-HCl, pH 8,4, 50 mM KCl, 1,5 mM $MgCl_2$, 0,2 mM dNTP (je 0,2 mM dATP, dCTP, dGTP, dTTP) je 50 pmol KC29 und KC43, 1 ng pKCN als Matrize und 2,5 U Tag-Polymerase vermischt. Der Ansatz wurde für 20 Zyklen (94°C, 40 sek; 40°C, 1 min; 72°C, 1 min) durchgeführt. Nach Beendigung der Reaktion wurde das amplifizierte Fragment gereinigt, mit *Bsp*MI und *Bam*HI hydrolysiert und in das mit *Nco*I/*Bam*HI gespaltene Plasmid pKE2 insertiert. Das Plasmid pKE2 enthält den *A. oryzae* alpha-Amylase-Promotor und den *A. nidulas trp*C-Terminator.

Die Konstruktion des Plasmids pKCN2 wurde durch eine Restriktionsanalyse und die anschließende Sequenzierung bestätigt.

Beispiel 2

Transformationsmethode für *Aspergillus*- und *Trichoderma ree*sei-Stämme

Von einer ca. zwei Wochen alten Petrischalenkultur des zu transformierenden Pilzstammes wurde eine Sporensuspension in ca. 10 ml 0,85 % NaCl durch Abschwemmen unter Zuhilfenahme eines Spatels hergestellt. Es wurden je vier 1 1-Schüttelkolben mit 100 ml Czapek-Dox-Minimalmedium (Oxoid) mit 0,1 % Hefeextrakt mit je 1 ml Sporensuspension beimpft und ca. 16 Stunden bei 28°C auf einem Rundschüttler bei 120 Umdrehungen pro Minute inkubiert. Das Mycel aus je vier Schüttelkolben wurde über einem Papierfilter geerntet und mit ca. 50 ml MP-Puffer (1,2 M $MgSO_4$ in 10 mM Phosphatpuffer, pH 5,8) gespült. Nach dem Ablaufen des Puffers wurde das feuchte Mycel gewogen. In der Regel wurden ca. 3 bis 5 g Feuchtmycel erhalten.

Pro g Feuchtmycel wurden 5 ml MP-Puffer, 120 $\mu$l Novozym-Lösung (1g Novozym® 234 (Novo Nordisk) in 6 ml MP-Puffer) und 25 $\mu$l β-Glucuronidase (Sigma) zugegeben. Die Mycel-Suspension wurde 5 min in Eiswasser gestellt. Anschließend wurden 60 $\mu$l Rinderserumalbumin-Lösung (0,2 g Rinderserumalbumin in 4 ml MP-Puffer, sterilfiltriert) zugegeben, und der Ansatz wurde unter leichtem Schütteln bei 30°C inkubiert. Die Bildung von Protoplasten wurde visuell im Mikroskop verfolgt. Wenn keine wesentliche Zunahme der Protoplastenbildung mehr festzustellen war, wurde der Ansatz zur Ernte der Protoplasten abgebrochen. Dies war in der Regel nach etwa 3 bis 5 Stunden der Fall.

Die Protoplastensuspension wurde zur Abtrennung noch vorhandener grober Mycelbestandteile über ein mit MP-Puffer getränktes Glaswollefilter gegeben und in Zentrifugenröhrchen überführt. Die obere Hälfte der Röhrchen wurde mit 600 mM Sorbit, 100 mM Tris/HCl, pH 7,0 überschichtet. Die Röhrchen wurden 10 min bei 2500 g zentrifugiert. Die Protoplasten wurden aus der Zwischenschicht abgenommen und in 1 M Sorbit, 10 mM Tris/HCl, pH 7,5 aufgenommen. Anschließend wurden die Protoplasten zweimal mit STC-Puffer (1 M Sorbit, 10 mM Tris/HCl, pH 7,5, 10 mM CaCl$_2$) durch Zentrifugation bei 1500 g gewaschen und zuletzt in 1 ml STC-Puffer aufgenommen.

Zur Transformation von *A. oryzae* wurden 300 μl Protoplastensuspension ca. 10 μg p3SR2 als Selektionsplasmid und 10 μg des jeweiligen Plasmids zur Expression der LPL in 25 μl 10 mM Tris/HCl pH 8,0 zusammengegeben und 10 min bei 0°C inkubiert. Anschließend wurden nochmals 25 μl des gleichen Plasmidgemisches und 400 μl PEG-Lösung (60% Polyethylenglykol 6000 (Fluka) in 10 mM Tris/HCl, pH 7,5, 50 mM CaCl$_2$) zusammengegeben, sehr vorsichtig vermischt und 5 min bei Raumtemperatur inkubiert. Es wurden nochmals 600 μl PEG-Lösung zugegeben, vermischt und der Ansatz für weitere 20 min bei Raumtemperatur inkubiert. Der Ansatz wurde mit ca. 9 ml Acetamid-Weichagar (Minimalmedium mit 10 mM Acetamid als einziger N-Quelle, 1 M Saccharose, 0,6 Gew.-% Agar) bei 45°C gemischt und auf vier Petrischalen mit dem gleichen Medium, jedoch mit 1,5 Gew.-% Agar (Oxoid) und zusätzlich 15 mM CsCl, verteilt. Die Platten wurden bei 28°C inkubiert. Nach 6 bis 10 Tagen wurden schnell wachsende Kolonien (Transformanten) auf Acetamid-Medium ohne Saccharose überimpft, zweimal über Einzelsporkolonien gereinigt und zuletzt auf Vollmedium, z.B. Kartoffel-Dextrose-Agar, übertragen.

Die Transformation von Stämmen der Arten *A. niger, A. awamori, A. japonicus* oder *A. foetidus* kann ebenfalls mit dem Plasmid p3SR2 erfolgen. Bevorzugt wurde die Transformation jedoch mit dem Plasmid pAN7-1 ausgeführt. Die Protoplastenpräparation und die Zugabe von Plasmid-DNA erfolgt dabei in analoger Weise, wie oben für das Plasmid p3SR2 beschrieben. Statt der Zugabe von Acetamid-Weichagar wird jedoch der gesamte Transformationsansatz zu 100 ml Czapek-Dox-Minimalmedium (Oxoid) mit 100 μg Hygromycin B/ml, 1,5 Gew.-% Agar (Oxoid) und 1 M Saccharose, abgekühlt auf ca. 45°C gegeben und vorsichtig vermengt. Der Ansatz wird dann in Portionen zu je 10 ml in Petrischalen gegeben, in denen jeweils 10 ml Czapek-Dox-Minimalmedium (Oxoid) mit 1,5 Gew.-% Agar (Oxoid), jedoch ohne Hygromycin und ohne Saccharose als feste Unterschicht vorgelegt war. Nach dem Erstarren der oberen Agarschicht werden die Petrischalen bei 30 bis 37°C inkubiert. Gegen Hygromycin B resistente Transformanten können nach ca. 3 bis 10 Tagen abgeimpft werden und zur Überprüfung der Resistenz auf Czapek-Dox-Minimalmedium (Oxoid) mit 50 μg Hygromycin B/ml und 1,5 Gew.-% Agar (Oxoid) übertragen werden.

Für die Transformation von *A. sojae* oder *A. phoenicis* wird ein drittes Selektionsprinzip genutzt, da die verwendeten Stämme dieser Art sowohl Acetamid verwerten wie auch gegen Hygromycin B resistent sind. Durch Selektion auf chlorathaltigem Nährboden werden Mutanten isoliert, deren Nitratreduktase-Gen (niaD) defekt ist, die also nicht mehr mit Nitrat als alleiniger Stickstoffquelle wachsen (Cove, D.J. (1976) Heredity 36, 191-203). Durch Transformation mit dem Plasmid pSTA10 (Unkles, S.E. et al. (1989) Mol.Gen.Genet. **218,** 99-104), auf dem sich die intakte Information für das Nitratreduktase-Gen befindet, wird der Defekt ausgeglichen, so daß die Transformanten mit Nitrat als alleiniger Stickstoffquelle wachsen, während die nicht transformierten Zellen im Wachstum zurückbleiben.

Diese Methodik zur Selektion ist außer für *A. sojae* genauso für andere *Aspergillus*-Arten geeignet; die Herstellung der niaD-Mutanten bedeutet allerdings einen zusätzlichen Arbeitsaufwand gegenüber der Verwendung der Hygromycin B-Resistenz oder der Acetamidverwertung.

Beispiel 3

Herstellung von PL sezernierenden Transformanten

Transformanten von A. *niger, A. awamori, A. foetidus, A. carbonarius* und A. *ellipticus*

Protoplasten dieser Aspergillus-Arten wurden mit der in Beispiel 1 beschriebenen Methodik hergestellt und unter Verwendung des Plasmids pAN7-1 und jeweils einem der Plasmide pKC3, pKC9, pKC12 oder pKCN2 cotransformiert. Zur Regeneration der Protoplasten wird der Transformationsansatz wie oben beschrieben auf Hygromycin plattiert, die Transformanten werden von den Regenerationsplatten isoliert, gereinigt und in Schüttelversuchen unter Verwendung der folgenden Nährlösung

| | |
|---|---|
| Maltodextrin | 3,75 % |
| Maisquellpulver | 3,0 % |
| KH$_2$PO$_4$ | 1,0 % |
| K$_2$HPO$_4$ | 0,7 % |
| Triton X-100 | 0,10 % |

in Leitungswasser, 30 min bei 121°C sterilisiert,

auf Produktion von PL geprüft. Dazu wird die Biomasse der Schüttelkulturen abfiltriert und die Phospholipase-Aktivität (PLU) im Kulturfiltrat gemessen. Transformanten zeichnen sich durch gegenüber dem Wirtsstamm deutlich erhöhte Phospholipase-Aktivität aus.

Transformanten von *A. oryzae* und *A. aculeatus*

Die Protoplastierung und Transformation wird auch mit diesen Arten wie im Beispiel 2 beschrieben durchgeführt. Die Protoplasten werden unter Verwendung des Plasmids p3SR2 und jeweils einem der Plasmide pKC3, pKC9, pKC12 oder pKCN2 cotransformiert. Zur Regeneration der Protoplasten wird der Transformationsansatz wie oben beschrieben auf Nährboden mit Acetamid als alleiniger Stickstoffquelle plattiert, die Transformanten werden von den Regenerationsplatten isoliert, gereinigt und in Schüttelversuchen unter Verwendung der folgenden Nährlösung

| | |
|---|---|
| Maltodextrin | 3,75 % |
| Maisquellpulver | 3,0 % |
| $(NH_4)_2HPO_4$ | 0,5 % |
| Triton X-100 | 0,10 % |
| in Leitungswasser, 30 min bei 121°C sterilisiert, | |

auf Produktion von PL geprüft.

Transformanten von A. *sojae* und A. *phoenicis*

Die Stämme *A. sojae* RH 3782 *nia*D22 und *A. phoenicis* RH 3828 *nia*D, beides nach Cove (1976) hergestellte Mutanten von *A. sojae* RH 3782 und *A. phoenicis* RH 3828, werden in der nachfolgenden Nährlösung aus

| | |
|---|---|
| Glucose (MERCK) | 2 % |
| Malzextrakt (OXOID) | 0,5 % |
| Bacto-Pepton (DIFCO) | 0,025 % |
| deionisiertes Wasser | |
| pH-Wert auf 5,0 einstellen; Sterilisation: 30 min bei 121°C | |

angezogen. Aus dem Mycel werden nach der in Beispiel 1 beschriebenen Methodik Protoplasten gewonnen und diese mit pSTA10 als Selektionsplasmid und jeweils einem der Plasmide pKC3, pKC9 oder pKC12 cotransformiert. Zur Regeneration der Protoplasten wird der Transformationsansatz mit 9 ml Weichagar (osmotisch stabilisierend) bestehend aus

| | |
|---|---|
| 0,1 M Na-Phosphat-Puffer pH 6,0 | 15 ml |
| 1 M Saccharose (MERCK) | 10,28 g |
| Millipore-Wasser auf | 29,1 ml |
| Agar (OXOID) | 0,18 g (= 0,6 %) |
| 30 min Sterilisation bei 121°C, anschließend sterile Zugabe von: | |
| Salzlösung (7.14.2) | 0,6 ml |
| 1 M $NaNO_3$-Lösung | 0,3 ml |

gemischt und auf vier Agarplatten der gleichen Zusammensetzung, jedoch mit 1 % Agar hergestellt, verteilt. Nach etwa 6 bis 10 Tagen Inkubation bei 37°C werden die Transformanten von den Agarplatten isoliert, auf Nitrat-Saccharose-Agar durch Ausstrich gereinigt. Es wurde eine Vielzahl Transformanten durch Selektion und anschließender Reinigung auf einem Nährboden mit Nitrat als einziger N-Quelle erhalten und in Schüttelkolben unter Verwendung der folgenden Nährlösung

| | |
|---|---|
| Maltodextrin | 3,75 % |
| Maisquellpulver | 3,0 % |
| $KH_2PO_4$ | 1,0 % |
| $K_2HPO_4$ | 0,7 % |

(fortgesetzt)

| | |
|---|---|
| Triton X-100 | 1,0 % |

in Leitungswasser, 30 min bei 121°C sterilisiert,

auf Produktion von PL geprüft.

Neben Transformanten, die keine oder nur geringe Mengen von PL produzieren, sowie die nicht transformierten Wirtsstämme, werden auch solche Transformanten gefunden, die deutlich erhöhte PL-Aktivität im Kulturfiltrat aufweisen. Diese als Cotransformanten bezeichneten Stämme eignen sich zur Herstellung des Enzyms. In der Tabelle 2 sind typische Ergebnisse der PL-Bildung durch Transformanten und durch die nicht transformierten Wirte gegenübergestellt.

Tabelle 2: Vergleich der PL-Bildung von Wirtsstämmen und Transformanten.

| Stamm bzw. Transformante | | relative PL-Aktivität |
|---|---|---|
| A. | *oryzae* RH 3745 | 100 |
| A. | *oryzae* RH 3745 p3SR2 pKC9 | 3000-4000 |
| A. | *oryzae* RH 3745 p3SR2 pKCN2 | 2000-2500 |
| A. | *sojae* RH 3782 *nia*D22 | 100 |
| A. | *sojae* RH 3782 *nia*D22 pSTA10 pKC9 | 500-700 |
| A. | *foetidus* RH 3046 | 100 |
| A. | *foetidus* RH 3046 pAN7-1 pKC9 | 1000-1500 |
| A. | *phoenicis* RH 3828 *nia*D | 100 |
| A. | *phoenicis* RH 3828 *nia*D pSTA10 pKC9 | 400-600 |
| A. | *ellipticus* | 100 |
| A. | *ellipticus* pAN7-1 pKC12 | 800-900 |
| A. | *heteromorphus nia*D | 100 |
| A. | *heteromorphus nia*D pSTA10 pKC9 | 900-1000 |
| A. | *carbonarius* | 100 |
| A. | *carbonarius* pAN7-1 pKC9 | 400-600 |
| A. | *aculeatus* | 100 |
| A. | *aculeatus* p3SR2 pKC9 | 900-1200 |
| A. | *niger* | 100 |
| A. | *niger* pAN7-1 pKC12 | 700-1000 |
| A. | *awamori* | 100 |
| A. | *awamori* pAN7-1 pKC12 | 600-800 |

Beispiel 4

Reinigung der PL aus *A. foetidus*

2080 ml Kulturretentat von *A. foetidus* RH 3788 wurden, um die elektrische Leitfähigkeit herabzusetzen, mit 3520 ml destilliertem Wasser verdünnt und mit 160 ml 1 M NaOH auf pH 7,0 eingestellt. Die Probe hatte ein Volumen von 5760 ml und eine Leitfähigkeit von 7,8 mS/cm.

In einem weiteren Schritt wurde eine Ionenaustauschchromatographie an DEAE-Fractogel (MERCK) vorgenommen. Dazu wurden 5760 ml der Enzymlösung in vier Ansätzen (à 1440 ml) auf eine DEAE-Fractogel-Säule (Höhe 278 mm, Durchmesser 100 mm) aufgetragen. Die Säule wurde mit Puffer A (20 mM Phosphatpuffer aus $Na_2HPO_4/KH_2PO_4$, pH 7,0 + 15 mM NaCl) gespült. Die Elution erfolgte in einem kontinuierlichen Gradienten von Puffer A zu Puffer B (Puffer A + 1 M NaCl). Es wurde bei einer Elutionsgeschwindigkeit von 70 ml/min eluiert und Fraktionen zu je 350 ml aufgefangen.

Die Fraktionen wurden auf Anwesenheit der PL getestet. Dies geschah durch Messung der PL-Aktivität. Eine PL-Einheit ist danach definiert als die Enzymmenge, die in einer wäßrigen Lösung von Lecithin bei pH 3,5 und 40°C eine Hydrolysegeschwindigkeit von 1 $\mu$M/min bewirkt.

Die Messung der PL-Aktivität wurde wie folgt ausgeführt:

Substrat: 1 g Epikuron 200 (Phosphatidylcholin von Fa. Lucas Meyer) + 100 g destilliertes Wasser + 5 ml 0,32

M CaCl$_2$-Lösung wurden mit dem Ultra Turrax homogenisiert.

Analyse: 10 ml Substrat wurden mit 10 ml 1 %iger Triton X-100 Lösung (Fa. Fluka) und 5 ml 0,0033 M Zitronensäure-Monohydratlösung versetzt und 10 min bei 40°C temperiert; der pH stellt sich auf 3,4 bis 3,5 ein. 0,1 ml Enzymlösung wurden zugegeben, und das Gemisch wurde 10 min bei 40°C inkubiert. Die Enzymkonzentration im Analysenansatz sollte nicht über 2,5 U/g liegen. Nach Ablauf der Reaktionszeit wurde mit 0,01 M KOH auf pH 10,0 zurücktitriert, wobei die ersten 5 ml zügig zugegeben wurden und dann die Titriergeschwindigkeit verringert wurde, um ein Übertitrieren zu vermeiden.

Für den Blindwert (BW) wurde das Enzym 15 min auf ca. 95°C erhitzt und inaktiviert. Nach dem Abkühlen verdünnte man es wie für den Hauptwert (HW) und verfuhr weiter wie mit dem Hautpwert.

```
Berechnung:

= PLU/g, pH 3,5 =  HW (ml) - BW (ml) * 0,01 M * 1000
                   10 min * 0,1 ml * Enzymkonz. g/ml
```

In der Patentanmeldung 195 27 274.9 vom 26.07.1995 wird die Phospholipase in Lecithase-Units, LU/g angegeben. 1 Lecithase-Unit ist dabei jene Enzymmenge, die bei 40°C, pH 8 aus Eigelb in einer Minute 1 $\mu$M Fettsäure freisetzt. 1 LU/g, pH 8 entsprechen 108 PLU/g, pH 3,5.

Die Elution der PL setzte bei ca. 0,11 M NaCl ein. Die PL-haltigen Fraktionen aus vier Läufen wurden vereinigt (8950 ml) und über einen CH2A-Konzentrator der Fa. Amicon, Hollow-Fiber Patrone MG10.000 auf ein Volumen von 2570 ml eingeengt. Diese Probe wurde mit 782 ml 3 M Ammoniumsulfatlösung unter Rühren versetzt (Probe enthält jetzt 0,7 M Ammoniumsulfat).

Im nächsten Schritt wurden 3352 ml der Probe auf eine Phenyl-Sepharose 6 Fast Flow, Low Substitution-Säule (Pharmacia, Höhe 215 mm, Durchmesser 100 mm) aufgetragen. Die Säule wurde mit Puffer C (20 mM Phosphatpuffer, pH 7,0 + 0,5 M Ammoniumsulfat) nachgewaschen und mit einem kontinuierlichen fallenden Gradienten von Puffer C nach Puffer D (20 mM Phosphat-puffer, pH 7,0) eluiert. Die PL-haltigen Fraktionen wurden vereint (790 ml) und mit dem Konzentrator (siehe oben) eingeengt und gegen Puffer D dialysiert; es wurden 150 ml Probe erhalten.

In einem weiteren Schritt wurden je 30 ml der Probe in 5 Ansätzen auf eine Mono Q-(Pharmacia, 6,3 ml)-Anionenaustausch-Chromatographie-Säule aufgetragen. Die Säule wurde mit Puffer D gespült, und die Elution erfolgte in einem kontinuierlichen Gradienten von Puffer D zu Puffer B und setzte dabei für die PL bei ca. 200 mM NaCl ein.

Die PL-haltigen Fraktionen wurden vereint und über PD-10-Säulen (Pharmacia) gegen Puffer E (20 mM Phosphatpuffer, pH 7,1) dialysiert. Die Probe hatte ein Volumen von 24 ml.

Endreinigung der PL über Mono P HR5/20 (Chromatofokussierung)

Die 24 ml (siehe oben) wurden auf die MONO P-Säule (Höhe 200 mm, Durchmesser 5 mm) aufgetragen. Die Säule wurde mit Puffer E nachgewaschen. Die Probe wurde mit Puffer F (Polybuffer 74 von Pharmacia 1:10 mit destilliertem Wasser verdünnt und auf pH 4,0 mit 1 M HCl eingestellt) eluiert. Die PL eluierte, nachdem das 13fache Säulenvolumen an Puffer F die Säule passiert hatte.

Das gereinigte Protein zeigt in der SDS-Gelelektrophorese eine einheitliche Bande mit einem Molekulargewicht von ca. 31000 Dalton. Der isoelektrische Punkt liegt bei ca. pH 4,3. Das auf diese Weise gereinigte Protein wurde zur Sequenzierung verwendet. Die so isolierte Phospholipase wurde zur Gewinnung von Antikörpern in Kaninchen verwendet. Die Immunisierung wurde mit dem bei Harlowe und Lane (Lit: Ed Harlowe und David Lane, Antibodies, Cold Spring Harbor Laboratory, 1988) beschriebenen Standardverfahren durchgeführt. Das erhaltene Antiserum konnte direkt für Western blots (wie ebenfalls bei Harlowe und Lane beschrieben) eingesetzt werden, wo es spezifisch die Phospholipasebande markierte.

Beispiel 5

Entschleimung von Sojaöl

200 g naßentschleimtes Sojaöl mit einem Rest-Phosphatgehalt von 160 ppm wird in einem Rundkolben auf 40°C erwärmt. Dazu gibt man 10 g Wasser, in dem 20 mg Zitronensäure und 100 Units Phospholipase enthalten sind. Das Enzym stammt aus der Fermentation einer *Aspergillus niger*-Transformante, die das Phospholipase-Konstrukt enthält. Die Aktivitätsbestimmung erfolgt bei pH 3,5. Dazu werden 10 ml 1 %iges Phosphatidylcholin (Epikuron 200 von Lucas Meyer), das 0,5 ml 0,32 M CaCl$_2$ enthält, mit 10 ml Triton X-100 Lösung und 5 ml 0,0033 M Zitronensäure-Monohydrat versetzt und 10 Minuten bei 40°C temperiert. 0,1 ml entsprechend verdünnter Enzymlösung werden zugegeben und 10 Minuten bei 40°C inkubiert. Mit 0,01 M KOH-Lösung wird auf pH 10 titriert. Der Blindwert (bei 95°C für 15 Minuten erhitzte Enzymlösung im Ansatz) wird abgezogen und die Berechnung gemäß Beispiel 3 vorgenommen.

Der Inhalt des Rundkolbens wird mittels einer externen Kreiselpumpe intensiv dispergiert. Dabei wird der Inhalt des Kolbens etwa einmal pro Minute durchgesetzt. Die Wasserphase liegt dabei in einer Teilchengröße von unter 1 μ. In Zeicabständen von zwei Stunden werden Proben genommen und auf Phosphorgehalt untersucht. Dabei ergaben sich folgende Werte:

| Zeit in Stunden | 0 | 2 | 4 | 6 | 8 |
|---|---|---|---|---|---|
| Phosphorgehalt in ppm | 160 | 24 | 12 | 7 | 3 |

Versuche, bei denen wie beschrieben verfahren wurde, aber anstelle des Enzympräparats eine entsprechende Menge Molkenprotein, also nichtenzymatisches Protein oder Lysophospholipase des Handels (G-Zyme von Enzyme Biosystems, USA, 1000 Lysophospholipase Units pro 200 ml Sojaöl) zugesetzt wurden, konnte der Phosphorgehalt nicht unter 80 ppm gesenkt werden.

Beispiel 6

Verbesserung der Teigqualität

Der folgende Backversuch wurde mit der erfindungsgemäßen Phospholipase durchgeführt. Aus 100 Gew. Tln. Mehl, 2 Gew. Tln. Salz, 3 Gew. Tln. Backhefe, 58 bis 60 Gew. Tln. Wasser und 40 bis 50 ppm Ascorbinsäure (bezogen auf das Teiggewicht) wurde in einem Spiralkneter (Fabrikat Kemper) 2 min auf niedriger Stufe 1 und 6 min auf höherer Stufe 2 ein Teig bereitet. Dem Wasser wurden vor Beginn des Knetvorgangs die Enzyme und anderen Zusätze zugegeben. Die Teigtemperatur betrug 23° bis 25°C. Nach einer Teigruhe von 20 min wurde der Teig zur Herstellung von freigeschobenem Weißbrot in 350 g-Stücke geteilt, geformt, 70 min bzw. 90 min bei 32°C und 80 % relativer Luftfeuchte gegart und 32 min bei 230°C gebacken. In der Tabelle 3 ist das Brotvolumen bei verschiedenen Enzymzusätzen angegeben. Die Backergebnisse zeigen, daß durch Zusatz von Phospholipase das Backvolumen und die Krumstruktur verbessert werden. Die stabilisierende Wirkung auf den Teig zeigt sich in den guten Backergebnissen bei verlängerter Garzeit (90 min).

Tabelle 3: Backversuche

| Zusätze/ 100 kg Mehl | Backvolumen 70 min Gare | % | 90 min Gare | % | Porenstruktur |
|---|---|---|---|---|---|
| ohne Zusatz | 1000 ccm | 100 | 1050 ccm | 100 | ungleichmäßig |
| Pilzamylase 10000 SKB | 1050 ccm | 105 | 1130 ccm | 107 | ungleichmäßig |
| Pilzamylase 10000 SKB + Phospholipase 2500 PLU | 1100 ccm | 110 | 1225 ccm | 117 | ungleichmäßig |
| Pilzamylase 50000 SKB | 1225 ccm | 122 | 1275 ccm | 121 | ungleichmäßig |
| Pilzamylase 50000 SKB + Phospholipase 12500 PLU | 1275 ccm | 128 | 1365 ccm | 130 | gleichmäßig |
| Pilz-Xylanase 12000 UXYL | 1325 ccm | 133 | 1375 ccm | 131 | gleichmäßig |
| Pilz-Xylanase 1200 UXYL + Phospholipase 12500 PLU | 1375 ccm | 138 | 1475 ccm | 140 | gleichmäßig |

**Patentansprüche**

1. Protein mit Phospholipaseaktivität erhältlich durch Spalten der reifen Sequenz der *Aspergillus*-Lysophospholipase mit SEQ ID NO:2 in zwei Spaltstücke, wobei die Spaltstücke über mindestens eine unter reduzierenden Bedingungen spaltbare Bindung verknüpft sind oder wobei von den unverknüpften Spaltstücken mindestens eines Phospholipaseaktivität besitzt.

2. Protein nach Anspruch 1, **dadurch gekennzeichnet, dass** eines der Spaltstücke ein Molekulargewicht von 30 kDa besitzt.

3. Protein nach einem der Ansprüche 1 oder 2, **dadurch gekennzeichnet, dass** die Spaltung der reifen Sequenz der *Aspergillus foetidus*-Lysophospholipase zwischen Position 44 und 45 der Aminosäuresequenz erfolgt ist.

4. Protein nach einem der Ansprüche 1 bis 3, **dadurch gekennzeichnet, dass** es aus *Aspergillus*-Kulturen isolierbar ist.

5. Protein nach Anspruch 4, **dadurch gekennzeichnet, dass** es aus Kulturen von *A. foetidus, A. niger* oder *A. oryzae* isolierbar ist.

6. Protein mit Phospholipaseaktivität nach Anspruch 1, **dadurch gekennzeichnet, dass** es von einem Antikörper gegen gereinigte Phospholipase aus *Aspergillus foetidus* RH 3046 erkannt wird.

7. Verfahren zur Produktion eines Proteins mit Phospholipaseaktivität nach Anspruch 1 durch Fermentation eines in geeigneter Weise transformierten Lysophospholipase produzierenden Wirtsorganismus in einem geeigneten Kulturmedium und Isolierung des Proteins mit Phospholipaseaktivität aus dem zellfreien Kulturfiltrat, **dadurch gekennzeichnet, dass** man die Fermentation im sauren bis leicht alkalischen Bereich durchführt.

8. Verfahren nach Anspruch 7, **dadurch gekennzeichnet, dass** man die Fermentation bei einem pH-Wert von 2 bis 9, bevorzugt 3 bis 8, durchführt.

9. Verfahren nach Anspruch 7 oder 8, **dadurch gekennzeichnet, dass** als transformierter Wirtsorganismus ein *Aspergillus*-Stamm oder ein *Trichoderma*-Stamm verwendet wird.

10. Verfahren nach Anspruch 9, **dadurch gekennzeichnet, dass** als transformierter Wirtsorganismus *Aspergillus foetidus* oder *Aspergillus oryzae* verwendet wird.

11. Verwendung eines Proteins nach einem der Ansprüche 1 bis 6 zur Entschleimung von pflanzlichem Öl.

12. Verwendung eines Proteins nach einem der Ansprüche 1 bis 6 als Backhilfsmittel.

## Claims

1. A protein having phospholipase activity, obtainable by cleaving the mature sequence of *Aspergillus*-lysophospholipase having SEQ ID NO: 2 into two cleavage products, wherein said cleavage products are linked via at least one bond which is cleavable under reducing conditions, or wherein at least one of said unlinked cleavage products has phospholipase activity.

2. The protein according to claim 1, **characterized in that** one of said cleavage products has a molecular weight of 30 kDa.

3. The protein according to any of the claims 1 or 2, **characterized in that** the cleavage of the mature sequence of the *Aspergillus foetidus*-lysophospholipase has occurred between position 44 and 45 of the amino acid sequence.

4. The protein according to any of the claims 1 to 3, **characterized in that** it is isolatable from *Aspergillus* cultures.

5. The protein according to claim 4, **characterized in that** it is isolatable from cultures of *A. foetidus, A. niger* or *A. oryzae.*

6. The protein having phospholipase activity according to claim 1, **characterized in that** it is recognized by an antibody against purified phospholipase from *Aspergillus foetidus* RH 3046.

7. A process for the production of a protein having phospholipase activity according to claim 1 by fermentation of a suitably transformed lysophospholipase producing host organism in a suitable culture medium and isolation of said protein with phospholipase activity from the cell-free culture filtrate, **characterized in that** said fermentation is carried out in the acidic to slightly alkaline range.

8. The process according to claim 7, **characterized in that** said fermentation is carried out at a pH value of from 2 to 9, preferably 3 to 8.

9. The process according to any of the claims 7 or 8, **characterized in that** a *Aspergillus* strain or a *Trichoderma* strain is used as a transformed host organism.

10. The process according to claims 9, **characterized in that** *Aspergillus foetidus* or *Aspergillus oryzae* is used a transformed host organism.

11. Use of a protein according to any of the claims 1 to 6 for degumming vegetable oil.

12. The use of a protein according to any of the claims 1 to 6 as a baking aid.

## Revendications

1. Protéine ayant une activité phospholipase pouvant être obtenue par clivage en deux produits de clivage de la séquence pure d'une lysophospholipase dérivée *d'Aspergillus* contenant SEQ ID N°2, les produits de clivage étant liés par l'intermédiaire d'au moins une liaison clivable dans des conditions réductrices ou au moins l'un des produits de clivage non liés possédant une activité phospholipase.

**2.** Protéine selon la revendication 1, **caractérisée en ce que** l'un des produits de clivage possède un poids moléculaire de 30 kDa.

**3.** Protéine selon la revendication 1 ou 2, **caractérisée en ce que** le clivage de la séquence pure de la lysophospholipase *Aspergillus foetidus* s'opère entre la position 44 et la position 45 de la séquence d'acides aminés.

**4.** Protéine selon l'une des revendications 1 à 3, **caractérisée en ce qu'**elle peut être isolée à partir de cultures de *Aspergillus.*

**5.** Protéine selon la revendication 4, **caractérisée en ce qu'**elle peut être isolée à partir de cultures de *A. foetidus, A. niger* ou *A. oryzae.*

**6.** Protéine ayant une activité phospholipase selon la revendication 1, **caractérisée en ce qu'**elle a été identifiée par un anticorps dirigé contre une phospholipase purifiée issue de *Aspergillus foetidus* RH 3046.

**7.** Procédé de production d'une protéine ayant une activité phospholipase selon la revendication 1, par fermentation dans un milieu de culture approprié d'un organisme hôte producteur d'une lysophospholipase et transformé de façon adéquate, et isolation de la protéine ayant une activité phospholipase à partir du filtrat de culture dépourvu de cellules, **caractérisé en ce que** l'on effectue la fermentation dans la plage acide à légèrement alcaline.

**8.** Procédé selon la revendication 7, **caractérisé en ce que** la fermentation est effectuée à un pH compris entre 2 et 9, de préférence entre 3 et 8.

**9.** Procédé selon la revendication 7 ou 8, **caractérisé en ce que** l'on utilise une souche *Aspergillus* ou une souche *Trichoderma* en tant qu'organisme hôte transformé.

**10.** Procédé selon la revendication 9, **caractérisé en ce que** l'on utilise *Aspergillus foetidus* ou *Aspergillus oryzae* en tant qu'organisme hôte transformé.

**11.** Utilisation d'une protéine selon l'une des revendications 1 à 6, pour la démucilagination d'une huile végétale.

**12.** Utilisation d'une protéine selon l'une des revendications 1 à 6, en tant qu'auxiliaire de panification.

FIGUR 1: Prozessierung des Lysophospholipasegens aus
Aspergillus und Erhalt der Phospholipase

LPL

SVSTS.................................EASTTMLLF...................................................

"leichte" Kette        "schwere " Kette

- im SDS-Gel mit und ohne Reduktion: ca. 36 kDa

- nur eine Sequenz

PL ohne Reduktion

SVSTS...............................

EASTTMLLEF.........................................................

"leichte" Kette

"schwere " Kette

- im SDS-Gel mit Reduktion ca. 30 kDa und ohne Reduktion: ca. 36 kDa

- Doppelsequenz, Verhältnis 1:1

PL mit Reduktion

EASTTMLLEF.............................................................

"leichte" Kette

"schwere " Kette

- im SDS-Gel ca. 30 kDa, leichte Kette läuft mit der Front daher nicht nachweisbar
- nur eine Sequenz

"schwere " Kette ca. 30 kDa

"leichte" Kette ca. 6 kDa

Summe ca. 36 kDa

FIGUR 2: Konstruktion des Plasmids pKCN2

SEQUENZPROTOKOLL

(1) ALLGEMEINE ANGABEN:

(i) ANMELDER:

(A) NAME: ROEHM GMBH

(B) STRASSE: Kirschenallee

(C) ORT: Darmstadt

(E) LAND: Deutschland

(F) POSTLEITZAHL: 64293

(A) NAME: LOEFFLER, Fridolin

(B) STRASSE: Karl-Henkelmann-Weg 4

(C) ORT: Bensheim

(E) LAND: Deutschland

(F) POSTLEITZAHL: 64625

(A) NAME: JUNGSCHAFFER, Gerald

(B) STRASSE: Haehnleiner Strasse 1A

(C) ORT: Alsbach-Haehnlein

(E) LAND: Deutschland

(F) POSTLEITZAHL: 64665

(A) NAME: KHANH, Quoc Nguyen

(B) STRASSE: Am Tannenberg 9

(C) ORT: Reichelsheim

(E) LAND: Deutschland

(F) POSTLEITZAHL: 64385

(A) NAME: SCHUSTER, Erwin

(B) STRASSE: Darmstaedter Str. 237

(C) ORT: Bensheim-Auerbach

(E) LAND: Deutschland

(F) POSTLEITZAHL: 64625

(A) NAME: SPROESSLER, Bruno

(B) STRASSE: Auf der Schmelz 93

(C) ORT: Rossdorf

(E) LAND: Deutschland

(F) POSTLEITZAHL: 64380

(A) NAME: WOLF, Sabine

(B) STRASSE: Otzbergstrasse 44

(C) ORT: Otzberg

(E) LAND: Deutschland

(F) POSTLEITZAHL: 64853

(ii) BEZEICHNUNG DER ERFINDUNG: Protein mit Phospholipaseaktivitaet

(iii) ANZAHL DER SEQUENZEN: 4

(iv) COMPUTER-LESBARE FASSUNG:

(A) DATENTRÄGER: Floppy disk

(B) COMPUTER: IBM PC compatible

(C) BETRIEBSSYSTEM: PC-DOS/MS-DOS

(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 1368 Basenpaare

(B) ART: Nucleotid

(C) STRANGFORM: Doppelstrang

(D) TOPOLOGIE: linear

(ii) ART DES MOLEKÜLS: Genom-DNA

(iii) HYPOTHETISCH: NEIN

(iv) ANTISENSE: NEIN

(ix) MERKMAL:

    (A) NAME/SCHLÜSSEL: intron

    (B) LAGE:222..275

(ix) MERKMAL:

    (A) NAME/SCHLÜSSEL: intron

    (B) LAGE:442..486

(ix) MERKMAL:

    (A) NAME/SCHLÜSSEL: intron

    (B) LAGE:824..874

(ix) MERKMAL:

    (A) NAME/SCHLÜSSEL: CDS

    (B) LAGE:join(140..221, 276..441, 487..823, 875..1180)

(ix) MERKMAL:

    (A) NAME/SCHLÜSSEL: mat_peptide

    (B) LAGE:221..1180

(ix) MERKMAL:

    (A) NAME/SCHLÜSSEL: sig_peptide

    (B) LAGE:140..220

(xi) SEQUENZBESCHREIBUNG: SEQ ID NO: 1:

```
ATGGGGAATT GGGGTGGGTA ATATGATACA GGTATAAAAG GGGGCTCGGA GGTGCAGTTG          60

GATAGAAGCA TTGTGTGTGC ATTGCAGCAG TCCGTTGGTC TCACGTCTCT GGTTGCCTCG         120

ATTGTATATA TACTGCAGG ATG TTC TCT GGA CGG TTT GGA GTG CTT TTG ACA         172
                      Met Phe Ser Gly Arg Phe Gly Val Leu Leu Thr
                      -27     -25                     -20

GCG CTT GCT GCG CTG TGT GCT GCG GCA CCG ACA CCA CTT GAT GTG CGG A        221
Ala Leu Ala Ala Leu Cys Ala Ala Ala Pro Thr Pro Leu Asp Val Arg
    -15             -10                     -5

GTAGGTGTGC CTGATTTGAA GTGGCTGGAT AGCACTGATG AAGGTTTTGA ATAG   GT         277
                                                              Ser
                                                              1

GTC TCG ACT TCC ACG TTG GAT GAG CTG CAA TTG TTC TCG CAA TGG TCT         325
Val Ser Thr Ser Thr Leu Asp Glu Leu Gln Leu Phe Ser Gln Trp Ser
            5               10              15

GCC GCA GCT TAT TGC TCG AAC AAT ATC GAC TCG GAC GAC TCT AAC GTG         373
Ala Ala Ala Tyr Cys Ser Asn Asn Ile Asp Ser Asp Asp Ser Asn Val
        20              25              30

ACA TGC ACG GCC GAC GCC TGT CCA TCA GTC GAG GAG GCG AGC ACC AAG         421
Thr Cys Thr Ala Asp Ala Cys Pro Ser Val Glu Glu Ala Ser Thr Lys
    35              40                  45

ATG CTG CTG GAG TTT GAC CT  GTATGTTGCT CCAGTGAAAT GGATAGAACA            471
Met Leu Leu Glu Phe Asp Leu
50              55

CAGCTGATTG AATAG G ACA AAT AAC TTT GGA GGC ACA GCC GGT TTC CTG          520
                Thr Asn Asn Phe Gly Gly Thr Ala Gly Phe Leu
                            60                  65

GCC GCG GAC AAC ACC AAC AAG CGG CTC GTG GTC GCC TTC CGA GGC AGT         568
Ala Ala Asp Asn Thr Asn Lys Arg Leu Val Val Ala Phe Arg Gly Ser
        70              75              80

AGC ACC ATC AAG AAC TGG ATT GCT GAT CTC GAC TTC ATC CTG CAA GAT         616
Ser Thr Ile Lys Asn Trp Ile Ala Asp Leu Asp Phe Ile Leu Gln Asp
        85              90                  95
```

```
AAC GAT GAC CTC TGT ACT GGC TGC AAG GTT CAC ACT GGA TTC TGG AAG        664
Asn Asp Asp Leu Cys Thr Gly Cys Lys Val His Thr Gly Phe Trp Lys
100             105                 110                 115

GCA TGG GAA GCC GCT GCA GAC AAT CTG ACG AGC AAG ATC AAG TCC GCG        712
Ala Trp Glu Ala Ala Ala Asp Asn Leu Thr Ser Lys Ile Lys Ser Ala
                120             125                 130

ATG AGC ACG TAT TCG GGC TAT ACC CTC TAC TTC ACC GGG CAC AGC TTG        760
Met Ser Thr Tyr Ser Gly Tyr Thr Leu Tyr Phe Thr Gly His Ser Leu
            135                 140                 145

GGC GGC GCA TTG GCT ACA CTG GGA GCA ACG GTC TTG CGA AAT GAC GGT        808
Gly Gly Ala Leu Ala Thr Leu Gly Ala Thr Val Leu Arg Asn Asp Gly
            150                 155                 160

TAT AGC GTT GAA CTG GTGAGTGCTT CAGAGGGTGA TCATTAAACA GCCGGTTCTG        863
Tyr Ser Val Glu Leu
            165

ACAGTCAATA G TAC ACC TAT GGA TGT CCT CGA GTC GGA AAC TAT GCG CTG       913
            Tyr Thr Tyr Gly Cys Pro Arg Val Gly Asn Tyr Ala Leu
                170                 175                 180

GCC GAG CAC ATC ACC AGC CAG GGA TCT GGA GCG AAC TTC CCT GTT ACA        961
Ala Glu His Ile Thr Ser Gln Gly Ser Gly Ala Asn Phe Pro Val Thr
                185                 190                 195

CAC TTG AAC GAC ATC GTC CCC CGG GTG CCA CCC ATG GAC TTT GGA TTC       1009
His Leu Asn Asp Ile Val Pro Arg Val Pro Pro Met Asp Phe Gly Phe
            200                 205                 210

AGC CAG CCA AGT CCA GAA TAC TGG ATC ACC AGT GGC ACC GGA GCC AGT       1057
Ser Gln Pro Ser Pro Glu Tyr Trp Ile Thr Ser Gly Thr Gly Ala Ser
            215                 220                 225

GTC ACG GCG TCG GAT ATT GAA CTC ATC GAG GGA ATC AAT TCG ACG GCG       1105
Val Thr Ala Ser Asp Ile Glu Leu Ile Glu Gly Ile Asn Ser Thr Ala
230                 235                 240                 245

GGG AAT GCA GGC GAA GCA ACG GTG GAC GTT TTG GCT CAC TTG TGG TAC       1153
Gly Asn Ala Gly Glu Ala Thr Val Asp Val Leu Ala His Leu Trp Tyr
            250                 255                 260

TTT TTC GCA ATT TCA GAG TGT CTG CTA TAGCTTGGAC AGTCCGATGA            1200
Phe Phe Ala Ile Ser Glu Cys Leu Leu
            265                 270

AATAAGTGCG GAGAGAAAGT GTAAATAGTA ATTAAGTATA TATCAGGCAG AGAAGCAGTG    1260

GTGGTCAGAG AAGAAAGAGT GAGTCCCATT ACGTAGCAGA TAACCACGTG TGGAGGCGCT    1320

GTTCCTCCAC TTGCAGTTGC GGCCATCAAT CATATTCTTC TCCTTACT               1368
```

(2) ANGABEN ZU SEQ ID NO: 2:

    (i) SEQUENZKENNZEICHEN:

        (A) LÄNGE: 297 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 2:

```
Met Phe Ser Gly Arg Phe Gly Val Leu Leu Thr Ala Leu Ala Ala Leu
-27     -25             -20             -15
Cys Ala Ala Ala Pro Thr Pro Leu Asp Val Arg Ser Val Ser Thr Ser
    -10             -5                  1                   5
Thr Leu Asp Glu Leu Gln Leu Phe Ser Gln Trp Ser Ala Ala Ala Tyr
                10                  15                  20
Cys Ser Asn Asn Ile Asp Ser Asp Asp Ser Asn Val Thr Cys Thr Ala
            25                  30                  35
Asp Ala Cys Pro Ser Val Glu Glu Ala Ser Thr Lys Met Leu Leu Glu
        40                  45                  50
Phe Asp Leu Thr Asn Asn Phe Gly Gly Thr Ala Gly Phe Leu Ala Ala
    55                  60                  65
Asp Asn Thr Asn Lys Arg Leu Val Val Ala Phe Arg Gly Ser Ser Thr
70              75                  80                      85
Ile Lys Asn Trp Ile Ala Asp Leu Asp Phe Ile Leu Gln Asp Asn Asp
            90                  95                  100
Asp Leu Cys Thr Gly Cys Lys Val His Thr Gly Phe Trp Lys Ala Trp
            105                 110                 115
Glu Ala Ala Ala Asp Asn Leu Thr Ser Lys Ile Lys Ser Ala Met Ser
        120                 125                 130
Thr Tyr Ser Gly Tyr Thr Leu Tyr Phe Thr Gly His Ser Leu Gly Gly
    135                 140                 145
Ala Leu Ala Thr Leu Gly Ala Thr Val Leu Arg Asn Asp Gly Tyr Ser
150                 155                 160                 165
Val Glu Leu Tyr Thr Tyr Gly Cys Pro Arg Val Gly Asn Tyr Ala Leu
            170                 175                 180
Ala Glu His Ile Thr Ser Gln Gly Ser Gly Ala Asn Phe Pro Val Thr
            185                 190                 195
His Leu Asn Asp Ile Val Pro Arg Val Pro Pro Met Asp Phe Gly Phe
        200                 205                 210
Ser Gln Pro Ser Pro Glu Tyr Trp Ile Thr Ser Gly Thr Gly Ala Ser
    215                 220                 225
Val Thr Ala Ser Asp Ile Glu Leu Ile Glu Gly Ile Asn Ser Thr Ala
230             235                 240                 245
Gly Asn Ala Gly Glu Ala Thr Val Asp Val Leu Ala His Leu Trp Tyr
            250                 255                 260
Phe Phe Ala Ile Ser Glu Cys Leu Leu
            265                 270
```

(2) ANGABEN ZU SEQ ID NO: 3:

    (i) SEQUENZKENNZEICHEN:

(A) LÄNGE: 42 Basenpaare
(B) ART: Nucleotid
(C) STRANGFORM: Einzelstrang
(D) TOPOLOGIE: linear

(ii) ART DES MOLEKÜLS: Genom-DNA
(iii) HYPOTHETISCH: NEIN
(iv) ANTISENSE: NEIN
(xi) SEQUENZBESCHREIBUNG: SEQ ID NO: 3:

GGAATTCACC TGCTAACCAT GTTCTCTGGA CGGTTTGGAG TG          42

(2) ANGABEN ZU SEQ ID NO: 4:

(i) SEQUENZKENNZEICHEN:

(A) LÄNGE: 34 Basenpaare
(B) ART: Nucleotid
(C) STRANGFORM: Einzelstrang
(D) TOPOLOGIE: linear

(ii) ART DES MOLEKÜLS: Genom-DNA
(iii) HYPOTHETISCH: NEIN
(iv) ANTISENSE: NEIN
(xi) SEQUENZBESCHREIBUNG: SEQ ID NO: 4:

CGGGATCCAA GCTATAGCAG ACACTCTGAA ATTG          34